# EUROPEAN PATENT APPLICATION

(11) **EP 4 778 496 A1**
(43) Date of publication of application: **22.07.2026**
(21) Application number: 25152135.7
(22) Date of filing: 15.01.2025
(51) Int. Cl.: A61F 2/24

(54) **STENT WITH DRY PERICARDIUM**

(71) Applicant: P+F Products + Features GmbH, 1190 Wien (AT)
(72) Inventor: AGRELI, Guilherme, Vienna (AT)
(74) Representative: Manitz Finsterwald Patent- und Rechtsanwaltspartnerschaft mbB

(57) **Abstract**

The invention relates to an expandable stent (10) that is expandable from an undeployed state into a deployed state and that in the deployed state comprises a stent frame (12) forming a body (16), with the body (16) having a height (H_{b}) extending from an inlet (I) to an outlet (O) of the expandable stent (10) in an axial direction (A) and with the body (16) circumferentially extending in a circumferential direction (C) around to the axial direction (A), a unidirectional valve member (38) arranged within the body (16) in the region of the outlet (O), the valve member (38) consisting of dry bovine pericardium with the dry bovine pericardium being configured to be rehydrated with a solution, and with the dry bovine pericardium having a calcium content selected in the range of 0.01 to 0.1 g/Kg, a plurality of fingers (18; 18') that is arranged in the region of the inlet (I) with the plurality of fingers (18, 18') projecting radially outwardly from the body (16) in a radial direction (B) and with the plurality of fingers (18; 18') forming at least part of an upper end (U) of the expandable stent (10, 10') together with the inlet (I), and a plurality of anchors (22; 22', 22") that is arranged in the region of the outlet (O), with a first end (24) of each of the plurality of anchors (22; 22', 22") projecting radially outwardly from the body (16) in the radial direction (B) in the region of the outlet (O) and with the first ends (24) of the plurality of anchors (22; 22', 22") forming at least part of a lower end (L) of the expandable stent (10, 10') together with the outlet.

## Description

The invention relates to an expandable stent that is expandable from an un-deployed state into a deployed state and that in the deployed state. The invention further relates to a method of assembling an expandable stent and to a method of treating biological tissue as well as to the use of a biological tissue.

A healthy heart facilitates oxygenated blood flow to the extremities. The heart is comprised of four chambers: the right and left sides, which manage deoxygenated and oxygenated blood respectively. Deoxygenated blood from the upper and the lower extremities, travels through the caval veins into the right atrium. It is pumped through the tricuspid valve and into the right ventricle. During systole, blood is pumped from the right ventricle, through the pulmonary valve and into the lungs. Following oxygenation, blood is pumped back to the left side of the heart through the pulmonary vein. Oxygenated blood flows through the mitral valve into the left ventricle. During systole, the left ventricle ejects the blood through the aortic valve to the rest of the body.

Proper opening and closing of the atrioventricular valves is dependent on the function of all of the structures involved with leaflet function, specifically the annulus, the leaflets, the chordae tendinae, the papillary muscles and a healthy myocardial wall. During ventricular filling (diastole), the atrioventricular valves remain open and during ventricular contraction (systole), the valves close as a consequence of complete leaflet apposition. In instances where complete apposition of the leaflets is not achieved, leakage or regurgitation across the valve affects cardiac performance and health. Severe regurgitation or leakage can lead to hemodynamic deterioration and/or heart failure.

Moderate or higher tricuspid valve regurgitation affects cardiac functional performance, and is typically indicative of a broader primary disease such as left heart dysfunction. Primary dysfunction can include pulmonary hypertension, right ventricular volume overload and right ventricular disease. Similarly, mitral regurgitation or leakage is primarily due to degenerative valve disease. The presence of dysfunctional support structures also contributes to mitral regurgitation, specifically as a consequence of a heart attack (ischemic mitral regurgitation) or ventricular dilation (functional mitral regurgitation). Other causes of atrioventricular regurgitation include acquired conditions (i.e. endocarditis, rheumatic heart disease) and congenital anomalies.

Atrioventricular regurgitation also impacts the caval veins leading to the atrium. During tricuspid regurgitation, pressure and flow changes in the right atrium causes the inferior vena cava (IVC) and the superior vena cava (SVC) to dilate. Additionally, veins feeding the IVC and the SVC are also impacted by these effects. Specifically, tricuspid regurgitation causes the azygos and the hepatic veins, which are located cranial and caudal to the right atrial opening respectively, to dilate. This leads to overall hemodynamic deterioration and liver dysfunction. Physical symptoms of tricuspid regurgitation include fatigue, loss of appetite and abdominal fullness. Similarly, mitral regurgitation impacts the pulmonary vein and can cause fluid buildup in the lungs. Chronic mitral regurgitation often leads to left ventricular remodeling or dilation of the left ventricle. Physical symptoms of mitral regurgitation include fatigue, decreased exercise tolerance, shortness of breath, and swollen feet. If left untreated, either atrioventricular severe regurgitation can lead to pulmonary hypertension, atrial fibrillation or heart failure.

On use of transcatheter valves to treat e.g. mitral disease, these modify the anatomy of the structures surrounding the mitral valve, deforming the aortic valve or lead to an occlusion of the coronary arteries or veins. A deformation of anatomical structures can lead to a malfunction of the heart, including the aortic valve. Other complications brought about by an implantation of a replacement mitral valve is a change in the flow properties of the blood exiting the mitral valve due to the design of the replacement valve or thrombus formation in the atrial portion respectively. Moreover, if the atrioventricular valve is not placed correctly, this cannot seal the ventricles from the atria correctly such that the underlying pathology is not treated correctly, creating a paravalvular leakage.

For this reason, it is an object of the present invention to provide a medical implant or prosthesis that can provide relief from tricuspid valve leakage or regurgitation and from mitral valve leakage or regurgitation. It is a further object of the present invention to minimize the deformation of anatomical structures and to ensure simple and reliable placement at the native valve in question. It is yet a further object of the present invention to minimize the recovery time of a patient following the treatment with such a medical implant or prosthesis.

These objects are satisfied by an expandable stent having the features in accordance with claim 1.

Such an expandable stent that is expandable from an un-deployed state into a deployed state and that in the deployed state comprises:
- a stent frame forming a body, with the body having a height extending from an inlet to an outlet of the expandable stent in an axial direction and with the body circumferentially extending in a circumferential direction around the axial direction,
- a unidirectional valve member arranged within the body in the region of the outlet, the valve member consisting of dry bovine pericardium with the dry bovine pericardium being configured to be rehydrated with a solution, and with the dry bovine pericardium having a calcium content selected in the range of 0.01 to 0.1 g/Kg,
- a plurality of fingers that is arranged in the region of, preferably directly at, the inlet, with the plurality of fingers projecting radially outwardly from the body in a radial direction and with the plurality of fingers forming at least part of, preferably a complete, upper end of the expandable stent together with the inlet, and
- a plurality of anchors that is arranged in the region of the outlet, with a first end of each of the plurality of anchors projecting radially outwardly from the body in the radial direction in the region of the outlet and with the first ends of the plurality of anchors forming at least part of, preferably a complete, lower end of the expandable stent together with the outlet.

The provision of an expandable stent permits the use of minimally invasive techniques reducing the recovery time of a patient following surgery. Hence, the stent for an implant or prosthesis described herein is in particular suitable for the treatment of mitral valve diseases via minimally invasive transcatheter implantation to replace a defective mitral valve.

In this connection it should be noted that the lower end of the expandable stent can only be formed by the plurality of anchors and the outlet. It should further be noted that the upper end of the expandable stent can only be formed by the plurality of fingers and the inlet. In this way on deployment of the replacement valve structure the lower and upper end can be used to clamp the native atrial wall in order to produce a replacement valve that is adapted to an e.g. native mitral valve and no components of the atrioventricular valve project into the respective ventricle.

By providing a stent frame that has no structures that project beyond the upper end or the lower end respectively into the flow path of the blood pumped through the respective valve during diastole, a distortion or alteration of the blood flow pattern is avoided.

Moreover, the replacement valve is significantly shorter than prior art stents in which the valve member is typically arranged at the center of the stent frame and not in the region of the outlet and in which structures of the stent frame thus also project into the space of the respective ventricle. This is presently avoided by forming the outlet and the inlet of the valve as part of the respective upper and lower ends of the atrioventricular valve.

In this connection it should be noted that it is preferred if tips of leaflets forming the valve member lie in a plane comprising the outlet and the plurality of fingers, i.e. in a plane at least essentially perpendicular to a flow of blood from the inlet through the mitral valve via the outlet into the left ventricle.

Preferably the plurality of anchors forms an atrial connection face and the plurality of fingers forms a ventricular connection face such that in use the mitral valve can clamp an atrial wall between the atrial connection face and the ventricular connection face so that a short length mitral valve does not project beyond the opening of the native mitral valve. In this way the flow properties of the blood exiting the mitral valve are not altered considerably.

Moreover, a pericardium as an animal biological tissue material is used, in particular obtained from a bovine heart that may have been treated with a crosslinking agent. The natural human heart valve, which is supposed to be fixed with the invention, is identified as the mitral valve. Therefore, the pericardium is used to replace the damaged or diseased naturally occurring heart valve. Also, such a valve can allow the pre-loading of the stent within a delivery system.

The valves are made using bovine pericardium. The ECM (Extracellular Matrix) tissue is generally harvested from the pericardial sac of cows and is then used to manufacture the leaflets. The tissue from pericardial sac is particularly well suited for a valve leaflet due to its durable physical properties. The tissues are glutaraldehyde fixed, non-viable, chemically treated (decellularized) and sterilized so that the biological markers are removed making them more compatible with the patient's immune system.

The potential benefits of bovine pericardium are superior biocompatibility, demonstrates minimal suture line bleeding and patency can be immediately confirmed by ultrasound, such as TEE ultrasound. They also have benefits like lack of calcification, support of cellular ingrowth and reduced rates of restenosis and infection. The pericardium is durable, strong and available in various sizes.

Also, the dry bovine pericardium may have a calcium content selected in the range of 0.01 to 0.1 g/Kg. The bovine leaflets may generally be as flexible and durable similar to the patient's natural tissue and therefor individual with such replacement valve may not require blood thinner medication on a continuous basis. Bovine pericardium tissue provides better hemodynamics in view of their similarity to natural flexible leaflet valves, some bovine pericardium valves may have some limitation on durability due to calcification and degeneration process. Treating the valves with a specialized anti-calcification treatment makes them more resistant to calcification. The valves having such a calcium content are hence more resistant to calcification and are more durable.

It is preferred if the stent frame at least substantially has the shape of an inner bearing race, thereby an as short as possible stent frame can be made available.

Advantageously a height of the body is selected in the range of 0.3 to 2.0 cm between the outlet and the inlet. These dimensions reflect a thickness of the native atrial wall.

It is preferred if an overall height of the atrioventricular valve between a lowest point of the lower end, i.e. the inlet, and an uppermost point of the atrioventricular valve, i.e. the plurality of fingers, is selected in the range of 0.3 to 2.5 cm. In this way, particularly short atrioventricular valves are made available that are similar in geometry to the native geometry and that do not alter the flow of blood through the valve into the respective ventricle.

In this way the height of the atrioventricular valve is selected such that it accommodates the unidirectional valve member within its body with at least one end of the unidirectional valve member ending at the outlet.

Preferably the plurality of anchors that form the lower end radially project away from the outlet towards the upper end. Such anchors can maintain a position of the native leaflets and thereby also enhance the function of a replacement atrioventricular valve.

Preferably the plurality of fingers and the plurality of anchors are arranged such that they at least generally project in the same axial direction. In this way the atrioventricular valve is adapted to the native shape of the atrial wall separating e.g. the left atrium from the left ventricle of a mammalian heart.

It should be noted in this connection that a cross-section of the lower end in the axial direction can have the shape of a truncated cone.

Advantageously a second end of the plurality of anchors extends over a height of the body. Thereby the body of the stent frame is reinforced in the regions having the second ends of the plurality of anchors.

Advantageously at least some of the plurality of anchors is configured as respective commissures for the valve member that is longitudinally attached to the plurality of anchors, preferably to the second ends thereof forming the commissures, and over the height of the body and circumferentially within the body in the region of the inlet, so that an upper surface of the valve member is present in the region of the inlet and preferably forms part of the upper end.

These commissures are then present in the reinforced regions of the stent frame enabling a long lifetime of such a replacement valve. Moreover, by attaching the valve member in this way leaks between the valve member and the body can be avoided.

Preferably the valve member comprises at least two leaflets, and in particular comprises four leaflets. It has hitherto been found that the use of four leaflets in an atrioventricular valve of elliptical design leads to a surgical valve that performs like a well-functioning native valve.

It is preferred if the stent further comprises a plurality of elongate struts and the plurality of elongate struts, the plurality of anchors and the plurality of fingers are formed from the same material, such as nitinol. In this way a one-piece stent frame can be provided that is made from a flexible self-expanding material.

It is preferred if the plurality of elongate struts, the plurality of anchors and the plurality of fingers have at least approximately the same thickness in the radial direction, and with the plurality of anchors and the plurality of fingers respectively having a greater width than the plurality of elongate struts in a circumferential direction of the body.

It is preferred if the plurality of fingers form a part of an inlet flange of the expandable stent forming the upper end. Likewise, it is advantageous if the plurality of anchors forms a part of an outlet flange of the expandable stent forming the lower end. The use of flanges as an upper and lower end can prevent the formation of leaks between the atrial and ventricular portion of the heart in the region of the replacement valve, as the flanges can respectively form a seal that seals off the upper and lower ends. Moreover, the inlet flange can form the atrial connection face and the outlet flange can form the ventricular connection face such that in use the mitral valve can clamp an atrial wall between the atrial connection face and the ventricular connection face.

Preferably the plurality of fingers and the plurality of anchors - and hence also the outlet and inlet flanges - act as cooperating clamps of the expandable stent. In this way cardiac tissue can be clamped between the plurality of fingers and the plurality of anchors to ensure a tight fit of the replacement atrioventricular valve.

Advantageously the plurality of fingers is formed by or extends from at least some of the plurality of elongate struts. Thereby a particularly simple design of the stent frame can be achieved.

Preferably at least some of the plurality of fingers comprises eyelets for a fixation of the expandable stent. Likewise, at least some of the plurality of anchors can comprise openings or apertures for a fixation of the expandable stent.

It is preferred if the plurality of anchors is formed by or extends from at least some of the material forming the body. Likewise, the plurality of anchors can comprise a first group of anchors and a second group of anchors, with a respective anchor of the first group of anchors being shorter than a respective anchor of the second group of anchors. Similarly, the plurality of fingers can be formed by or extend from at least some of the material forming the body and can also comprise a first group of fingers and a second group of fingers, with a respective finger of the first group of fingers being shorter than a respective finger of the second group of fingers.

It should be noted in this connection that at least some of the fingers of one of the groups of fingers can comprise eyelets and/or that at least some of the anchors of one of the groups of anchors can comprise openings, whereas the respective other group of fingers or anchors respectively can include no eyelets or no openings.

It is preferred if the body has an at least generally elliptical shape in a cross-section perpendicular to the axial direction. The selection of an elliptical shape means that the implant is configured similar to the native mitral valve and thereby avoids a deformation of the native anatomy and thereby does not influence the heart in an unpredictable manner.

It is even more preferable if the body has an at least generally cylindrical outer shape over its height in the axial direction. This enables a smooth fit of the body to the native valve.

Preferably a diameter of the body at the inlet is greater than or approximately the same as a diameter of the body at the outlet. In this way the mitral valve can be designed in accordance with the anatomical shape of the mammalian heart.

It is preferred if the expandable stent further comprises anti-leakage material, with the anti-leakage material being arranged to cover at least part of an inner and/or an outer surface of the body, preferably with the anti-leakage material being arranged to at least substantially completely cover the inner and/or the outer surface of the body, optionally wherein the anti-leakage material at least partly covers the plurality of fingers that form the upper end and/or the plurality of anchors that form the lower end.

In this way unwanted leaks between e.g. the left atrium and the left ventricle can be avoided ensuring an improved function of the replacement mitral valve.

The dry bovine pericardium may have a maximum tensile stress selected in the range of 20 to 25 MPa, and/or wherein the rehydrated bovine pericardium has a tensile stress selected in the range of 12 to 15 MPa. Thus, the dry bovine pericardium can comprise a tensile resistance which can be up to 15 times higher than the tensile resistance of the leaflets of a human heart. This is mainly done for safety reasons in order to minimize tearing or fracture of the pericardium.

The mechanical properties of a material, in particular tensile strength, can be tested under strain-stress evaluation using Universal Testing Machine (Oswaldo Filizzola, model AME-2kN).

Also, the dry bovine pericardium may further **be characterized by one or more of the following properties:**
**A calcium Content:** Selected in the range of 0.01 to 0.1 g/kg, thereby ensuring reduced calcification risk and improved long-term durability; and/or
**A collagen Composition:** Primarily type I collagen fibers, with a highly organized structure contributing to mechanical strength and durability; and/or.
**A thickness:** Typically ranges between 0.3 to 0.5 mm, providing a balance between flexibility and structural integrity; and/or
**A Tensile Stress:** The dry bovine pericardium has a maximum tensile stress of 20 to 25 MPa, while the rehydrated bovine pericardium exhibits a tensile stress of 12 to 15 MPa, ensuring adequate mechanical resistance under dynamic cardiac loading.
**A tear Resistance:** Tested using standard strain-stress protocols, the material exhibits sufficient tear resistance to withstand cardiac cycle pressures without structural failure.
**A moisture Content (Dry State):** Below 1%, ensuring stability during storage and reducing microbial contamination risk.
**Crosslinking and Treatment:** The tissue is chemically stabilized using glutaraldehyde, followed by an anti-calcification treatment with EDTA and ethanol/glycerol solutions to minimize mineralization.
**Biocompatibility:** The pericardium is decellularized to remove cellular components while preserving the collagen matrix, improving biocompatibility and reducing the risk of immune response.

The invention further relates to method of assembling an expandable stent comprising the steps of:
(1) providing bovine pericardium as biological tissue as a heart valve;
(2) providing a stent frame;
(3) attaching the heart valve comprising the biological tissue to the stent frame;
(4) testing the functionality of the biological tissue at the stent frame; and
(5) carrying out a method of decellulizing the biological tissue.

Decellularized pericardium offers several critical advantages for transcatheter valve manufacturing, including reduced immune rejection by removing cellular antigens, decreased calcification risk by eliminating nucleation sites, and preserved mechanical integrity with tensile strength values of 20-25 MPa (dry) and 12-15 MPa (rehydrated). The extracellular matrix, particularly type I collagen, is maintained, ensuring flexibility and durability for cyclic loading in the cardiovascular system. Additionally, the tissue's compatibility with anti-calcification treatments like EDTA and glycerol enhances long-term performance. These properties make decellularized pericardium an ideal material for durable, biocompatible, and effective transcatheter valves.

The invention further relates to a method of treating biological tissue comprising the steps of:
(1) soaking of the biological tissue treated with a crosslinking agent with a saline solution;
(2) contacting the soaked biological tissue with an aqueous solution comprising Hydrogen Peroxide;
(3) contacting the biological tissue with an aqueous solution comprising PBS and EDTA;
(4) contacting the biological tissue with a solution comprising glycerol, ethanol and EDTA; and
(5) contacting the biological tissue with a glycerol solution.

One embodiment of the present invention utilizes soaking of the bovine pericardium treated with a crosslinking agent with a saline solution.

As used herein, a crosslinking agent is glutaraldehyde which is preferably used in biochemical and medicine applications as an amine-reactive homobifunctional crosslinker. Glutaraldehyde treatment produces stable cross-links in cellular and extra-cellular matrix proteins which substantially reduced graft immunogenicity. However, such tissue has altered mechanical properties, early mechanical failure, cytotoxicity, and incomplete suppression of immunological recognition. Besides this severe calcification was noticed in glutaraldehyde-treated bovine pericardium. An emerging alternative to glutaraldehyde treatment is further treatment according to the method steps, i.e. a method allowing to reduce calcification of the bovine pericardium.

It is preferably to use the crosslinking agent in an amount of from 0.1% to 5.0 % by volume, more preferably from 0.2% to 3.0% by volume, further preferably from 0.3% to 2.0% by volume and especially preferably from 0.5% to 1.0% by volume.

In this respect, as a first step a soaking of the bovine pericardium with an aqueous saline solution comprising 0.9% of sodium chloride (9.0 g per litre) is carried out. Such a solution is also commonly named as normal saline, physiological saline or isotonic saline solution.

In a second step, the soaked bovine pericardium is contacted with an aqueous solution comprising Hydrogen Peroxide. It is preferred that the concentration of hydrogen peroxide is from 0.05% by volume to 5.0% by volume, preferably from 0.1% by volume to 3.0% by volume, more preferably from 0.2% by volume to 2.0% by volume.

In a third step of the present invention, the bovine pericardium is contacted with an aqueous solution comprising PBS and EDTA.

As used herein, the term "contacting" means treating, immersion, exposing to, rinsing of the biological tissue used in the inventive method.

As used herein, the term "PBS" is directed to a phosphate buffered saline having a pH of 7.4 and containing a water based salt solution of disodium hydrogen phosphate, sodium chloride and, in some formulations, potassium chloride and potassium dihydrogen phosphate. PBS is used in biological and medical applications, such as washing cells, transportation of tissues and dilutions, because PBS closely mimics the pH, osmolarity, and ion concentrations of the human body.

The term "aqueous solution" refers to a solution comprising a substance or a compound and water that has been purified to remove contaminants which are able to influence the end product. Preferably, distilled water, double distilled water or deionized water is used in a method of the present invention.

The term "EDTA" is used herein to refer to ethylenediaminetetraacetic acid which is a complexing chelating agent being able to sequester metal ions especially like Fe²⁺/Fe³⁺, Al³⁺, Mg²⁺, Ca²⁺, Mn²⁺, Zn²⁺ and others and to remove them from the solution forming so called EDTA-complexes.

According to embodiment, it is especially important to remove calcium ions from the solution by forming calcium chelator that has been shown to inhibit mineralization of biological tissues, in particular bovine pericardium tissue. It is suggested that EDTA binds to calcium ions on the outer shell of hydroxyapatite crystals which are formed from calcium phosphate crystals thereby chelating and removing calcium ions from the crystals, causing the tissue material to shrink thus demineralizing the material.

Treatment of biological tissues with EDTA hence slows down the progression of calcification by binding calcium before it can react to form hydroxyapatite. Since the calcification of biological tissues used e.g. as bioprosthetic heart valves is a clinically significant problem that contributes to implant failure, it is of significant importance to reduce calcium level in biological tissues used as an implant. Therefore, in the present invention, the EDTA treatment can reduce calcium level in biological tissues, especially in bovine or porcine pericardium or a heart valve preferably by 20%, more preferably by 30%, further preferably by 40% and especially preferably by 50%. Further, it is preferable to use EDTA in combination with PBS in order to increase demineralization and compatibility with a human body.

Furthermore, it is preferable to use EDTA, in particular in steps (3) and (4), having a concentration of more than 0.01% by weight, preferably of more than 0.05% by weight, more preferably of more than 0.10% by weight, still preferably of more than 0.15% by weight, and of less than 10.0% by weight, preferably of less than 8.0% by weight, more preferably of less than 6.0% by weight, still preferably of less than 5.0% by weight, further preferably of less than 3.0% by weight. Still further in the present invention, it is preferably to use disodium EDTA.

In a fourth step of the present invention, the bovine pericardium is contacted with a solution comprising glycerol, ethanol and EDTA, and in a fifth step the bovine pericardium is contacted with a glycerol solution in order to further reduce calcification of biological tissue and to dehydrate the bovine pericardium. The following steps describe an implementation of these processes in the method.

After the bovine pericardium has been processed through steps (1) to (3) of the method, they undergo the treatment in a solution comprising glycerol, ethanol and EDTA.

Phospholipids in and around biological tissue cells have been found the most prominent calcification nucleation sites. Therefore, the removal of these tissue components has been proposed to reduce mineralization, in particular calcification. Different studies have shown these to be effective calcification prevention strategies. The organic solvents like ethanol or glycerol or a mixture of ethanol and glycerol can be similarly used for this purpose. For example, the treatment with at least 70% ethanol, preferably with at least 80% ethanol, more preferably with at least 90% ethanol, extracts phospholipids from the tissue while also causing a change in collagen conformation that increases bioprosthesis resistance to collagenase. Thus, ethanol treatment allows extracting almost all phospholipids and cholesterols from the bioprosthesis, thus eliminating calcification of the biological tissue cells. Additionally, ethanol treatment also prevents adsorption of phospholipids and cholesterols from the solution. The method by which glycerol fixes biological tissue is not jet fully understood, but a 98% concentration, preferably 99% concentration, is sufficient to treat the biological tissue to make the tissue more biocompatible and resistant to calcification.

In this respect, it is preferably to treat biological tissue in a solution comprising glycerol, ethanol and EDTA for at least 60 minutes, preferably for at least 75 minutes, more preferably for at least 90 minutes, at room temperature, in particular at a temperature of 10°C to 25°C, preferably at a temperature of 15°C to 25°C, more preferably at a temperature of 18°C to 22°C, under stirring of not more than 500 rpm, preferably of not more than 300 rpm, more preferably of not more than 50 rpm. During this time most of the water molecules presented in biological tissue, in particular pericardial tissue, are replaced with glycerol.

Furthermore, it is preferable to use a mixture of glycerol and ethanol, wherein a volume ratio of glycerol to ethanol is preferably from 1:5 to 5:1, more preferably from 1:4 to 4:1, still preferably from 1:3 to 3:1, further preferably from 1:2 to 2:1.

The bovine pericardium is then removed from the solution and placed in glycerol for further dehydration for at least 60 minutes, preferably for at least 75 minutes, more preferably for at least 90 minutes, at room temperature, in particular at a temperature of 10°C to 25°C, preferably at a temperature of 15°C to 25°C, more preferably at a temperature of 18°C to 22°C, under stirring of not more than 500 rpm, preferably of not more than 300 rpm, more preferably of not more than 50 rpm.

It can further be preferable to use an additional step of contacting or rinsing the bovine pericardium with ethanol having a concentration of at least 70% by volume, preferably with at least 80% by volume, more preferably with at least 90% by volume. The additional step, in particular step (3a), is preferably carried out before contacting the biological tissue with a solution comprising glycerol, ethanol and EDTA. It can further be preferable to carry out another additional step (5a) of contacting the biological tissue with ethanol after the step of contacting the biological tissue with a glycerol and before the step of drying the biological tissue. It can still further be preferable to carry out an additional step (3a) and/or (5a) using a mixture of ethanol and EDTA having a concentration as in step (3) or (4).

The bovine pericardium is removed from the solution and exposed to ambient air or an inert environment, e.g. nitrogen, at room temperature and humidity so as not to adversely affect tissue properties. Preferably, the drying is performed in a clean room at ambient conditions for at least 12 hours, preferably for at least 16 hours, still preferably for at least 20 hours. Further preferably, the drying is performed under high efficiency particulate air (HEPA) filter, in particular under HEPA conditions in a clean room. As used herein, the term "ambient conditions" is directed to the ambient temperature of more than 10°C, preferably of more than 12°C, more preferably of more than 14°C, especially preferably of more than 18°C, and preferably of less than 25°C, more preferably of less than 23°C, further preferably of less than 22°C. Further in the present invention it is preferably to carry out each of steps (1) to (7) at the ambient conditions as described above.

The treated and dried bovine pericardium is then packaged in a container or package essentially free of liquid for subsequent surgical implantation. As used herein, the term "essentially free of liquid" means a non-fluid environment in which the presence of water or other substances is limited to approximately the content of such substances in ambient air.

According to another aspect of the invention an expandable stent, in particular the expandable stent according to the invention and/or in particular obtainable by the method according invention, is provided comprising a biological tissue prepared by the method according to the invention.

The invention further relates to the use of a biological tissue prepared by the method according to the invention in an expandable stent according to the invention.

The advantages described in connection with the expandable stent correspond to those or are similar to those that can be obtained by means of the methods in accordance with the invention.

The invention will be described in detail by means of embodiments and with reference to the drawings. These show preferred deployment locations and embodiments of the valve. The features described may be configured in various combinations, which are encompassed in this document. The drawings show:
- Fig. 1: a side view of a first embodiment of a mitral valve having a first kind of stent frame;
- Fig. 2: a top view of the first embodiment of Fig. 1;
- Fig. 3: a photograph of a further embodiment of a mitral valve arranged within a heart;
- Fig. 4: a delivery device for deploying the mitral valve of Fig. 1 in a heart;
- Fig. 5: a further kind of stent frame;
- Fig. 6a & 6b: schematic views of a further kind of stent frame;
- Fig. 7a & 7b: schematic views of a further kind of stent frame;
- Fig. 8a & 8b: schematic views of a further kind of stent frame;
- Fig. 9a & 9b: schematic views of a further kind of stent frame; and
- Fig. 10: schematically, a system of cardiac valves installed in a mammalian heart.

The following detailed description is merely exemplary in nature and is not intended to limit the invention or the application and uses of the invention. Although the description of embodiments hereof is in the context of mitral heart valve replacement, the invention may be adapted to be used for other valve replacements such as a tricuspid valve replacement. Furthermore, any expression relating to direction or position in this application is made relative to the position of installation and/or to the position in the Figures.

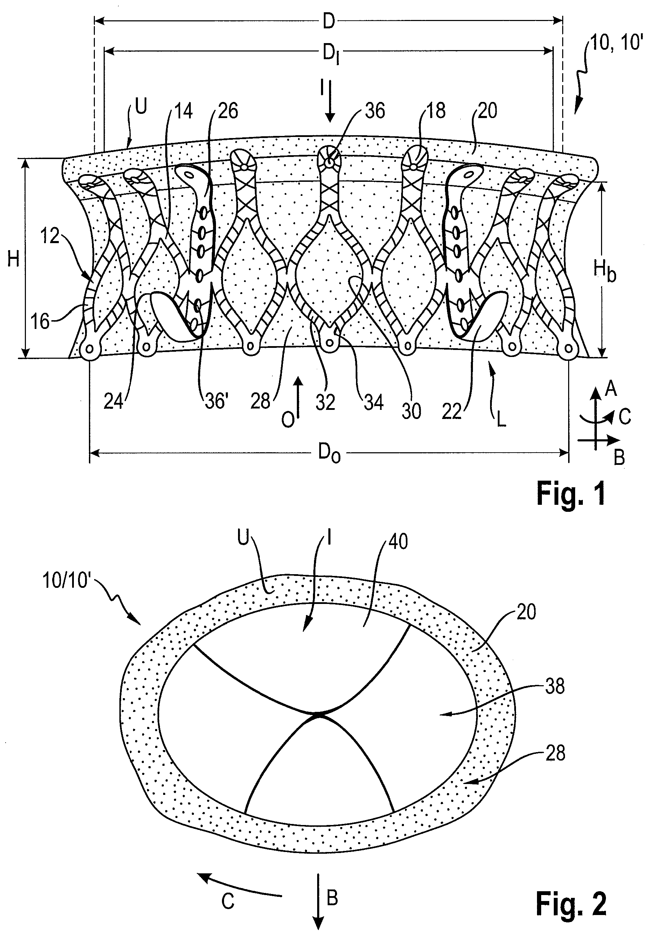

Fig. 1 shows a side view of a first embodiment of an expandable stent 10 configured as a mitral valve 10'. The mitral valve 10' is present in a deployed state, this means in an expanded state. The mitral valve 10' comprises a stent frame 12 formed from a plurality of elongate struts 14. A part of each of the plurality of elongate struts 14 forms a body 16 of the mitral valve 10'. A height H_{b} of the body 16 extends from an inlet I to an outlet O in an axial direction A.

A plurality of fingers 18 is arranged at the inlet I and forms an upper end U of the expandable stent 10 together with the inlet. Some of the plurality of fingers 18 is formed as extensions of the plurality of elongate struts 14 in the example shown in Fig. 1. The plurality of fingers 18 is radially deflected outwardly from the body 16 in the radial direction B directly at the inlet I. The plurality of fingers 18 thereby forms a part of an inlet flange 20 of the expandable stent 10 forming part of the upper end U of the mitral valve 10' together with the inlet I.

Furthermore, a plurality of anchors 22 is provided at the mitral valve 10'. A first end 24 of each of the plurality of anchors 22 projects radially outwardly from the body 16 in the region of the outlet O and forms a lower end L of the mitral valve 10' together with the outlet O. The first ends 24 of the plurality of anchors 22 also extends towards the upper end U.

The first ends 24 of the plurality of anchors 22 are formed by a flexible frame and are provided to capture leaflets of e.g. a native mitral valve and to keep the position of these natural leaflets close to the mitral valve 10' described herein.

A second end 26 of the plurality of anchors 22 extends in the axial direction A towards the inlet I along the height H_{b} of the body 16 in the form of bridges.

Typical heights H_{b} of such bodies 16 of such mitral valves 10' are selected in the range of 0.3 to 2 cm. In the present instance the body has a height H_{b} of 1.5 cm. The mitral valve 10' typically has an overall height H of 0.3 to 2.5 cm measured from the outlet O or lower end L respectively, i.e. the lowest point of the mitral valve 10', to an uppermost point of the mitral valve 10' present at the inlet flange 20. In the present instance the overall height H is 0.7 cm.

The general outer shape of the mitral valve 10' can be compared to that of an inner bearing race. This means that an outer contour of the mitral valve 10' has a region of enlarged outer diameter at the inlet I and defined by the plurality of fingers 18, a region of reduced outer diameter that is defined by an outer diameter of the body 16 and that is arranged between the inlet I and the outlet O and a region of enlarged outer diameter at the outlet O that is defined by the plurality of anchors 22.

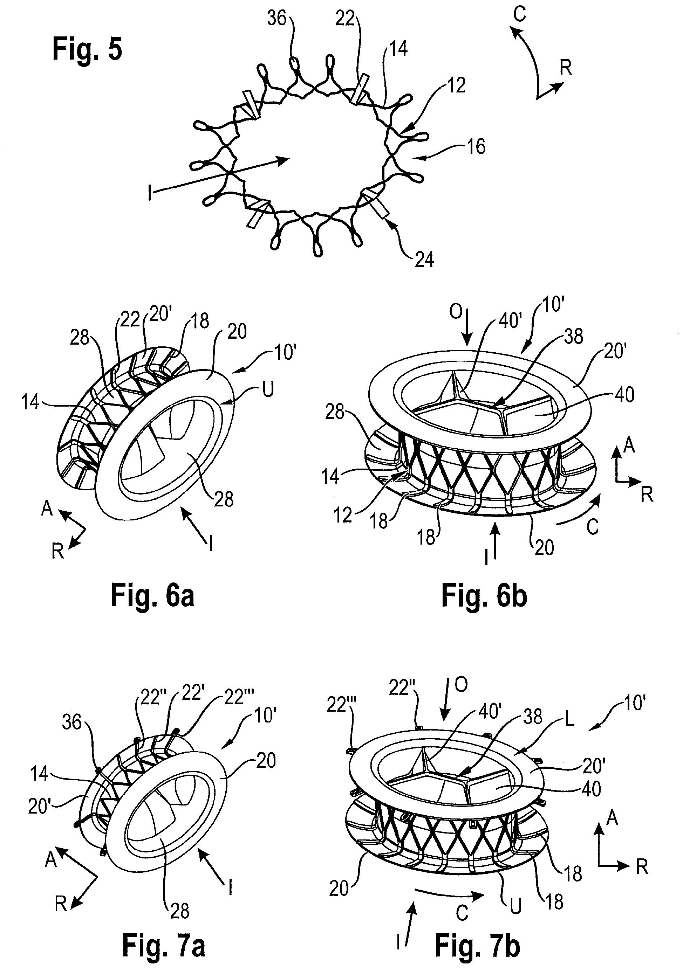

It should be noted in this connection that some of the plurality of fingers 18 are formed as extensions of the plurality of anchors 22 in the example of Fig. 1. It should further be noted in this connection that embodiments can exist in which the plurality of fingers 18 can be formed such that they only form extensions of at least some of the plurality of elongate struts 14 (see e.g. Fig. 5 in this regard).

An inner diameter Dₒ of the body 16 at the outlet O, i.e. the diameter of the outlet, is greater than an inner diameter Dᵢ of the body 16 at the inlet I, i.e. the diameter of the inlet. The body 16 has a generally planar inner surface 30 with an inner diameter D of the body 16 reducing over the height H_{b} of the body 16 from the outlet O to the inlet I.

In this connection it should be noted that the inner diameter Dₒ of the body 16 at the outlet O can also be equal to the inner diameter Dᵢ of the body 16 at the inlet I in such a way that the body 16 has an at least substantially like inner diameter D over the height H of the body 16.

Anti-leakage material 28 is affixed to the inner surface 30 of the body 16 and to an outer surface 32 of the plurality of fingers 18 by means of sutures 34. In order to improve the attachment of the anti-leakage material 28 to the stent frame 12, a plurality of eyelets 36 is provided at the plurality of fingers 18 and at the plurality of elongate struts 14. The bridges or second ends respectively also include apertures 36' therein.

It should be noted in this connection that the anti-leakage material 28 can also be attached to an outer surface of the body 16.

It should also be noted in this connection that the anti-leakage material 28 shown in Figs. 1 and 2 comprises woven material, can however likewise be formed by a dip coating process in which a coating is formed on the stent frame 12 by dipping the stent frame into a bath of coating material, a molding process in which a coating is molded to the stent frame 12 or by a spray process in which a coating of anti-leakage material is sprayed onto the stent frame 12.

The plurality of elongate struts 14, the plurality of fingers 18 and the plurality of anchors 22 are formed from the same flexible material namely nitinol in the present instance. The plurality of elongate struts 14, the plurality of fingers 18 and the plurality of anchors 22, have at least approximately the same thickness in the radial direction. Moreover, the plurality of fingers 18 and the plurality of anchors 22 respectively have a greater width than the plurality of elongate struts in a circumferential direction C of the body 16.

Fig. 2 shows a top view of the first embodiment of Fig. 1 onto the upper end U of the mitral valve 10' having the inlet I formed therein. A unidirectional valve member 38 arranged is within the body 16 at the inlet I. The valve member 38 shown in the present example comprises four leaflets 40. It should be noted in this connection that tips of the leaflets 40 (not shown) lie in the plane of the outlet (also not shown).

In this connection it should be noted that blood flows from the inlet I through the unidirectional valve member 38 of the mitral valve 10' via the outlet O into the left ventricle.

It should further be noted in this connection that the four leaflets 40 of the valve member 38 are respectively connected to the anti-leakage material 28 covering the inner surface 30 of the body 16. To this end the four leaflets 40 are circumferentially connected to the anti-leakage material in the region of the inlet. Moreover, the leaflets are also longitudinally attached to the second ends 26 of the plurality of anchors 22 that hence act as commissures.

The valve member 38, and especially the leaflets 40, is typically formed by biological tissue, i.e. by dry bovine pericardium. It could generally also be possible that the leaflets 40 would be formed by porcine pericardium, bacterial cellulose or another suitable material. Upon placement of the valve 10', the dry bovine pericardium is configured to be rehydrated with a solution, such as a saline solution. Furthermore, the dry bovine pericardium has a calcium content selected in the range of 0.01 to 0.1 g/Kg and a maximum tensile stress selected in the range of 20 to 25 MPa, and/or wherein the rehydrated bovine pericardium has a tensile stress selected in the range of 12 to 15 MPa.

The pericardium may further be characterized by one or more of the following features:
**Calcium Content:** Ranges from 0.01 to 0.1 g/Kg, ensuring minimal calcification risk.
**Tensile Strength (Dry):** Between 20 to 25 MPa, providing structural integrity and resistance to tearing during handling and implantation.
**Tensile Strength (Rehydrated):** Ranges from 12 to 15 MPa, balancing flexibility with durability once the pericardium is rehydrated prior to implantation.
**Collagen Content and Orientation:** The bovine pericardium used consists of highly organized collagen fibers, primarily type I collagen, which provides mechanical durability and minimizes leaflet deformation over time.
**Thickness:** The pericardium has a typical thickness ranging from 0.3 to 0.5 mm, ensuring optimal leaflet flexibility and hemodynamic performance.
**Crosslinking Treatment:** Treated with glutaraldehyde for structural stabilization, while anti-calcification agents (e.g., EDTA) are applied to further reduce mineralization.
**Biocompatibility:** The tissue is decellularized and chemically treated to minimize immunogenic response while preserving mechanical properties.
**Moisture Content (Dry State):** Less than 1% to ensure long-term preservation and prevent microbial growth during storage.

The number of leaflets 40 used in an atrioventricular valve 10 depends on the shape of the atrioventricular valve 10, for an atrioventricular valve 10 used as a mitral valve 10', an elliptical shape as currently shown has found to be advantageous. Moreover, elliptically shaped mitral valves 10' work best with four leaflets 40. It has namely been found that such a shape adapts best to the natural shape of a natural mitral valve (not shown). The coaptation (area of interference) and length of the leaflets 40 can be adjusted according to the performance criteria defined during manufacturing of the mitral valve 10'.

In this connection it should be noted that the plurality of anchors 22 are also configured as commissures for the leaflets 40 of the valve member 38. To this end the bridges or second ends 26 respectively include the apertures 36' (see Fig. 1) for sewing the leaflets 40 to the stent frame 12.

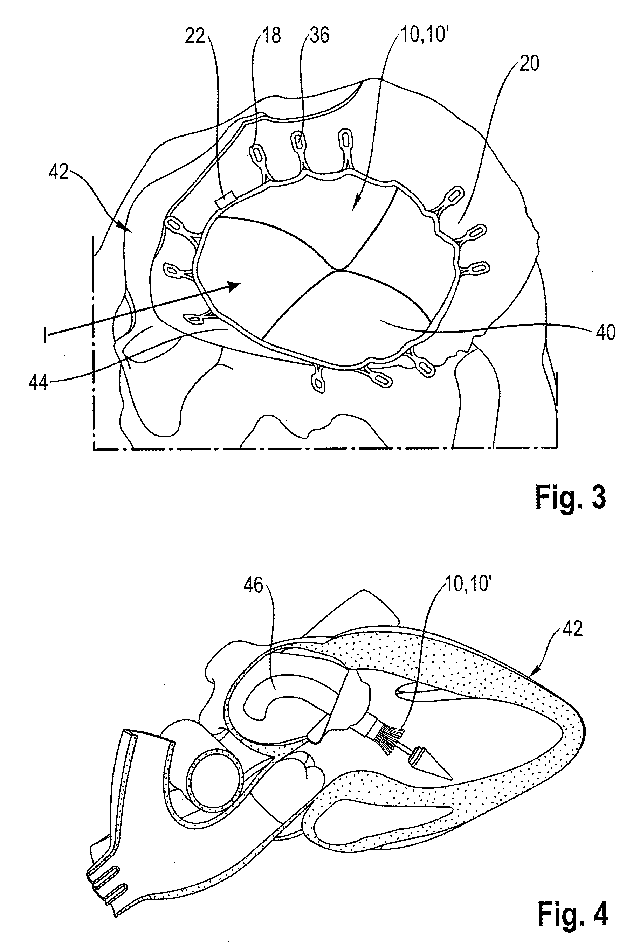

Fig. 3 is a photograph of a further kind of mitral valve 10' placed within a heart 42 as viewed from a left atrium. The body 16 of the mitral valve 10' and the plurality of fingers 18 are adapted to the anatomy of the native mitral valve ring in such a way that the plurality of fingers 18 sit on an atrial wall 44 of the native mitral wall ring, with the body 16 extending through the native mitral valve ring such that the native anatomical structure is deformed as little as possible. Moreover, the mitral valve 10' seals the previously present paravalvular leakage by means of the four leaflets 40, the inlet flange 20 and the anti-leakage material 28 (not shown).

Due to the deflection of the fingers radially outwardly, they clamp into position at the atrial wall 44 in the left ventricle. Moreover, no components of the mitral valve 10' project into the space of the left ventricle. Likewise, the plurality of anchors 22 clamp the atrial wall 44 on the atrial side of the native mitral valve ring valve in such a way that the plurality of fingers 18 and the plurality of anchors 22 act as cooperating clamps in the deployed state of the expandable stent.

Fig.4 shows a delivery device 46 for deploying the mitral valve 10' into the heart 42. In order to effect this the mitral valve 10' has distal connectors and proximal connectors (both not shown) that enable a connection to the delivery device 46. During minimally invasive surgery, the delivery device 46 transports the mitral valve 10' into the left atrium. The replacement mitral valve 10' is then placed approximate to the native mitral valve ring and deployed there. On deployment the valve is expanded from an un-deployed state into the deployed state. Thereby the plurality of anchors 22 fix one side of the atrial wall 44, whereas the plurality of fingers 18 clamp the other side of the atrial wall in order to clamp the atrial wall between the plurality of anchors 22 and the plurality of fingers 18. The delivery device 46 allows a controlled deployment at the position of the native mitral valve. It can also include additional possibilities to adjust the position of the mitral valve 10' during deployment.

In order to guide the delivery device 46 and to check the correct positioning of the mitral valve 10', this can include radiopaque markers that are preferably made of tantalum and indicate at least one of a position of the inlet flange 20, at least one of the plurality of anchors 22, at least one of a short and a long axis of an ellipse forming the mitral valve 10'.These radiopaque markers enable a tracking of the movement and position of the mitral valve 10' using e.g. x-rays.

Fig. 5 shows a further kind of stent frame 12. In this embodiment the plurality of anchors 22 only extend up to the inlet I of the body 16 of the stent frame 12. The first ends 24 of the plurality of anchors 22 are designed similar to those shown in Fig. 1 and have a generally rectangular shape.

Fig. 6a and 6b schematically show views of a further stent frame 12. The plurality of fingers 18 of this stent frame 12 do not comprise eyelets (see e.g. Fig. 1). Thereby the contact area of the plurality of fingers 18 to the anti-leakage material 28 and to the atrial wall 44 can be increased. Moreover, the anti-leakage material 28 also extends over a surface of the plurality of anchors 22 in the region of the outlet O to form an outlet flange 20'.

Tips 40' of the leaflets 40 lie in the plane of the outlet O, with the unidirectional valve member 38 dividing the valve member into the outlet O and the inlet I.

Figs. 7a & 7b schematically show views of a further kind of stent frame 12. The plurality of anchors of this stent frame 12 comprise a first group of anchors 22' and a second group of anchors 22". The first group of anchors 22' is provided for the attachment of the anti-leakage material 28 to the stent frame 12, whereas the second group of anchors 22" is provided for an attachment of the mitral valve 10' to the atrial wall 44. A respective anchor of the first group of anchors 18' is shorter than a respective anchor of the second group of anchors 18". Also a respective anchor of the first group of anchors 18' does not comprise an opening, whereas a respective anchor of the second group of anchors 22" does comprise an opening 22‴.

This design of stent frame 12 can also improve the contact area between both the anti-leakage material 28 and the stent frame 12 and the contact area between the stent frame 12 and the atrial wall 44. Moreover, one of the first and second group of anchors 22', 22" can comprise connectors (not shown) to the delivery device 46 of Fig. 4.

It should be noted in this connection that the inlet may also comprise more than one type of finger (not shown) that forms part of the inlet flange 20, with the types of fingers being arranged groupwise.

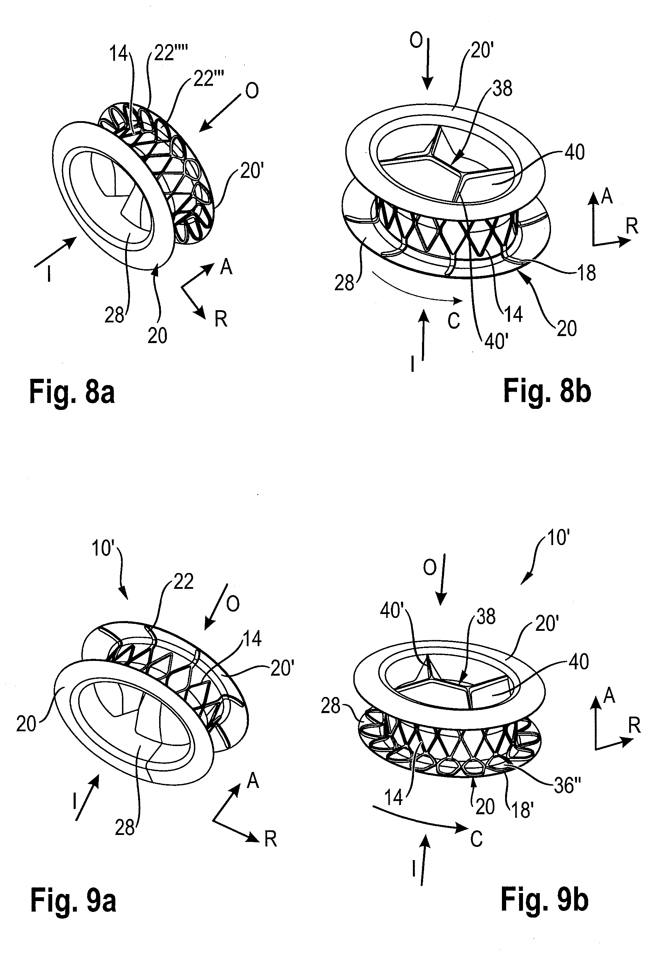

Figs. 8a & 8b schematically show views of a further stent frame 12 of this mitral valve 10'. This stent frame 12 comprises a plurality of arc shaped anchors 22ʺʺ that each comprise an opening 22". These arc shaped anchors 22ʺʺ increase the contact area between the anti-leakage material in the region of the outlet flange 20' and the stent frame 12.

Figs. 9a & 9b schematically show views of a further stent frame 12 for a mitral valve 10' which comprises a plurality of fingers 18' that are each formed of arc-shape and have an orifice 36" formed therein which hence acts as an attachment means in order to enable an improved connection of the plurality of fingers 18' to the anti-leakage material 28 and to the atrial wall 44 via the orifices 36".

The arc-shaped anchors 22' that form part of the outlet flange 20' provide an outlet flange 20' that is more rigid in comparison to the outlet flanges 20' of Figs. 6a to 8b. Such a rigid outlet flange 20' can have beneficial effects with regard to the durability of the mitral valve 10' as the pressure exerted on the mitral valve 10' is present at the outlet O and hence at the outlet flange 20'.

In this connection it should be noted that the further designs of both the plurality of fingers and the plurality of anchors can be provided at the inlet and outlet flanges respectively (not shown). The function of the plurality of fingers and of the plurality of anchors is to facilitate the connection of the valve to the atrial wall and/or to the anti-leakage material forming a seal between the different chambers of the heart.

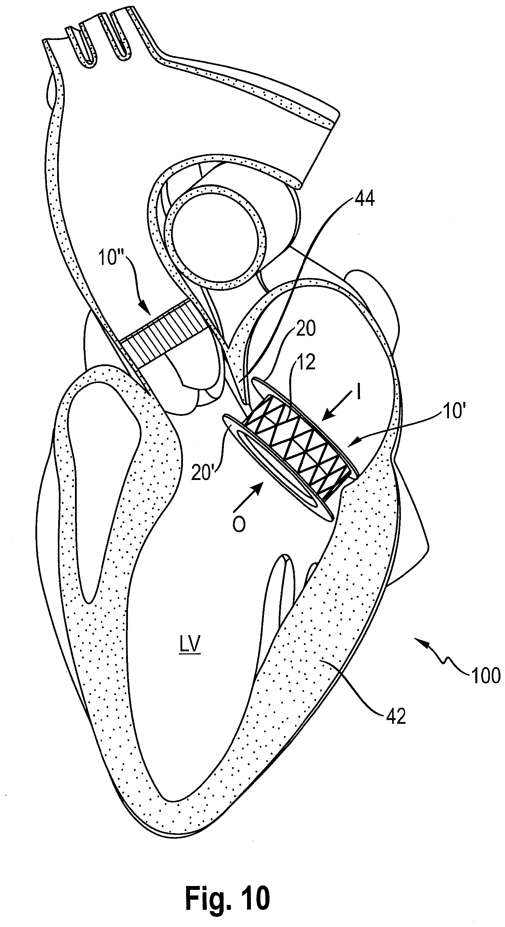

Fig. 10 shows a system of cardiac valves 100 comprising a mitral valve 10' and a replacement aortic valve 10". It has namely been found that once e.g. a mitral valve 10' has been positioned in the heart 42, the outflow properties of the mitral valve 10' are significantly enhanced in comparison to the defective native mitral valve and the downstream native aortic valve may have become accustomed to the behavior of the native mitral valve such that its performance needs enhancing following mitral valve replacement surgery.

### List of reference numerals:

- 10: atrioventricular valve
- 10': mitral valve
- 10": aortic valve
- 12: stent frame
- 14: elongate struts
- 16: body
- 18, 18': finger
- 20: inlet flange
- 20': outlet flange
- 22, 22ʺʺ: anchor
- 22', 22": groups of anchors
- 22‴: anchor opening
- 24: first end
- 26: second end
- 28: anti-leakage material
- 30: inner surface of body
- 32: outer surface of finger
- 32': outer surface of body
- 34: suture
- 36: eyelet
- 36': aperture
- 36": orifice of arc shaped finger 18'
- 38: valve member
- 40: leaflet
- 40': tip of leaflet
- 42: heart
- 44: atrial wall
- 46: delivery device
- 100: system of cardiac valves
- A: axial direction
- B: radial direction
- C: circumferential direction
- Dᵢ: inner diameter of the body at the inlet
- Dₒ: inner diameter of the body at the outlet
- D: inner diameter of the body
- H: height
- H_{b}: height of body
- I: inlet
- L: lower end
- LV: left ventricle
- O: outlet
- RA: right atrium
- RV: right ventricle
- U: upper end
The invention is further defined by the following embodiments:

An expandable stent (10) according to a first embodiment that is expandable from an un-deployed state into a deployed state and that in the deployed state comprises:
- a stent frame (12) forming a body (16), with the body (16) having a height (H_{b}) extending from an inlet (I) to an outlet (O) of the expandable stent (10) in an axial direction (A) and with the body (16) circumferentially extending in a circumferential direction (C) around to the axial direction (A),
- a unidirectional valve member (38) arranged within the body (16) in the region of the outlet (O), the valve member (38) consisting of dry bovine pericardium with the dry bovine pericardium being configured to be rehydrated with a solution, and with the dry bovine pericardium having a calcium content selected in the range of 0.01 to 0.1 g/Kg,
- a plurality of fingers (18; 18') that is arranged in the region of the inlet (I) with the plurality of fingers (18, 18') projecting radially outwardly from the body (16) in a radial direction (B) and with the plurality of fingers (18; 18') forming at least part of an upper end (U) of the expandable stent (10, 10') together with the inlet (I), and
- a plurality of anchors (22; 22', 22") that is arranged in the region of the outlet (O), with a first end (24) of each of the plurality of anchors (22; 22', 22") projecting radially outwardly from the body (16) in the radial direction (B) in the region of the outlet (O) and with the first ends (24) of the plurality of anchors (22; 22', 22") forming at least part of a lower end (L) of the expandable stent (10, 10') together with the outlet.

The stent according to embodiment 2, which may include all features of embodiment 1, wherein the stent frame (12) at least substantially has the shape of an inner bearing race.

The stent according to embodiment 3, which may include all features of embodiments 1 or 2, wherein the height (Hb) of the body (16) is selected in the range 0.3 to 2.0cm, and/or wherein an overall height (H) of the stent (10, 10') of the stent (10, 10') between an uppermost point of the upper end (U) and a lowermost point of the lower end (L) is selected in the range of 0.3 to 2.5cm.

The stent according to embodiment 4, which may include all features of the preceding embodiments, wherein the plurality of anchors (22; 22', 22") radially project away from the outlet (O) towards the upper end (U); and/or wherein the plurality of fingers (18; 18') and the plurality of anchors (22; 22', 22') are arranged such that they at least generally project in the same axial direction (A).

The stent according to embodiment 5, which may include all features of the preceding embodiments, wherein a second end (26) of the plurality of anchors (22) extends over the height (H_{b}) of the body (16) in the axial direction (A); and/or wherein at least some of the plurality of anchors (22) is configured as respective commissures for the valve member (38) that is longitudinally attached to the plurality of anchors (22), preferably to (the) second ends (26) thereof forming the commissures, over the height (H_{b}) of the body (16) and circumferentially within the body (16) in the region of the inlet (I), so that an upper surface of the valve member (38) is present in the region of the inlet (I) and preferably forms part of the upper end (U).

The stent according to embodiment 6, which may include all features of the preceding embodiments, wherein the valve member (38) comprises at least two leaflets (40), and preferably comprises four leaflets (40).

The stent according to embodiment 7, which may include all features of the preceding embodiments, further comprising a plurality of elongate struts (14) forming at least part of the body (16) of the stent frame (12), wherein the plurality of elongate struts (14), the plurality of fingers (18; 18') and the plurality of anchors (22; 22', 22") are formed from the same material, such as nitinol, have at least approximately the same thickness in the radial direction (B), and with the plurality of anchors (22; 22', 22") and the plurality of fingers (18; 18') respectively having a greater width than the plurality of elongate struts (14) in the circumferential direction (C) of the body (16).

The stent according to embodiment 8, which may include all features of the preceding embodiments, wherein the plurality of fingers (18; 18') form a part of an inlet flange (20) of the expandable stent (10, 10') forming the upper end (U) and/or wherein the plurality of anchors (22; 22', 22") form a part of an outlet flange (20') of the expandable stent (10, 10') forming the lower end (L).

The stent according to embodiment 9, which may include all features of the preceding embodiments, wherein the plurality of fingers (18; 18') and the plurality of anchors (22; 22', 22") act as cooperating clamps of the expandable stent (10, 10').

The stent according to embodiment 10, which may include all features of the preceding embodiments, wherein the plurality of anchors (22; 22', 22") is formed by or extends from at least some of the material forming the body (16).

The stent according to embodiment 11, which may include all features of the preceding embodiments, wherein the plurality of anchors comprise a first group of anchors (22') and a second group of anchors (22"), with a respective anchor of the first group of anchors (22') being shorter than a respective anchor of the second group of anchors (22").

The stent according to embodiment 12, which may include all features of the preceding embodiments, wherein the body (16) has an at least generally elliptical shape in a cross-section perpendicular to the axial direction (A).

The stent according to embodiment 13, which may include all features of the preceding embodiments, wherein the body (16) has an at least generally cylindrical outer shape over its height (H_{b}) in the axial direction (A).

The stent according to embodiment 14, which may include all features of the preceding embodiments, wherein a diameter (Dᵢ) of the body (16) at the inlet (I) is greater than or equal to a diameter (Dₒ) of the body (16) at the outlet (O) in a cross-section perpendicular to the axial direction (A).

The stent according to embodiment 15, which may include all features of the preceding embodiments, further comprising anti-leakage material (28), with the anti-leakage material being arranged to cover at least part of an inner and/or an outer surface (30, 32') of the body (14).

The stent according to embodiment 16, which may include all features of embodiment 15, wherein the anti-leakage material (28) is arranged to at least substantially completely cover the inner and/or the outer surface (30, 32') of the body (14).

The stent according to embodiment 17, which may include all features of the preceding embodiments 15 or 16, wherein the anti-leakage material (28) at least partly covers the plurality of fingers (18; 18') that form the upper end (U) and/or the plurality of anchors (22; 22', 22") that form the lower end (L).

The stent according to embodiment 18, which may include all features of the preceding embodiments, wherein the dry bovine pericardium has a maximum tensile stress selected in the range of 20 to 25 MPa, and/or wherein the rehydrated bovine pericardium has a tensile stress selected in the range of 12 to 15 MPa.

The invention further relates to a method of assembling a expandable stent according to a first embodiment comprising the steps of:
(1) providing bovine pericardium as the biological tissue as a heart valve;
(2) providing a stent frame;
(3) attaching the biological tissue to the stent frame;
(4) testing the functionality of the biological tissue at the stent frame; and
(5) carrying out a method of decellulizing the biological tissue.

The invention further relates to a method of treating biological tissue according to a first embodiment comprising the steps of:
(1) soaking of the biological tissue treated with a crosslinking agent with a saline solution;
(2) contacting the soaked biological tissue with an aqueous solution comprising Hydrogen Peroxide;
(3) contacting the biological tissue with an aqueous solution comprising PBS and EDTA;
(4) contacting the biological tissue with a solution comprising glycerol, ethanol and EDTA; and
(5) contacting the biological tissue with a glycerol solution.

The method according to embodiment 2, which may include all features of embodiment 1, wherein the method further comprises steps (3a) and/or (5a) of contacting the biological tissue with ethanol within a concentration of at least 70% by volume.

The method according to embodiment 3, which may include all features of the preceding embodiments 1 or 2, wherein the crosslinking agent is glutaraldehyde.

The method according to embodiment 4, which may include all features of the preceding embodiments, wherein the biological tissue is bovine or porcine pericardium or a heart valve.

The method according to embodiment 5, which may include all features of the preceding embodiments, wherein the saline solution is an aqueous solution comprising 0.9% of sodium chloride.

The method according to embodiment 6, which may include all features of the preceding embodiments, wherein a volume ratio of glycerol : ethanol in step (4) is from 1:5 to 5:1.

The method according to embodiment 7, which may include all features of the preceding embodiments, wherein EDTA in steps (3) and (4) has a concentration of 0.01% to 10.0 % by weight or wherein hydrogen peroxide in step (2) has a concentration of 0.05% to 5.0% by volume.

The method according to embodiment 8, which may include all features of the preceding embodiments, wherein the method further comprises the steps of (6) drying the biological tissue, (7) placing the biological tissue in a package, (8) sealing the package and (9) sterilizing the package.

The method according to embodiment 9, which may include all features of embodiment 8, wherein the sterilizing of the package comprising the biological tissue is carried out by exposing the package to ethylene oxide gas.

The method according to embodiment 10, which may include all features of the preceding embodiments, especially in accordance with claim 8, wherein the steps (1) to (5) are carried out at a temperature of 10°C to 25°C, especially wherein the steps (6) and (7) are carried out at a temperature of 10°C to 25°C.

The method according to embodiment 11, which may include all features of the preceding embodiments, wherein the steps (4) and (5) are carried out while stirring for at least 60 minutes.

The invention further relates to an expandable stent, in particular the expandable stent according to one of the embodiments 1 to 18 and/or in particular obtainable by the method according to the invention, comprising a biological tissue prepared by the method according to one of the embodiments 1 to 11.

Use of a biological tissue prepared by the method according to one of the embodiments 1 to 11 in an expandable stent according to any one of the embodiments 1 to 18.

## Claims

1. An expandable stent (10) that is expandable from an un-deployed state into a deployed state and that in the deployed state comprises:
- a stent frame (12) forming a body (16), with the body (16) having a height (H_{b}) extending from an inlet (I) to an outlet (O) of the expandable stent (10) in an axial direction (A) and with the body (16) circumferentially extending in a circumferential direction (C) around to the axial direction (A),
- a unidirectional valve member (38) arranged within the body (16) in the region of the outlet (O), the valve member (38) consisting of dry bovine pericardium with the dry bovine pericardium being configured to be rehydrated with a solution, and with the dry bovine pericardium having a calcium content selected in the range of 0.01 to 0.1 g/Kg,
- a plurality of fingers (18; 18') that is arranged in the region of the inlet (I) with the plurality of fingers (18, 18') projecting radially outwardly from the body (16) in a radial direction (B) and with the plurality of fingers (18; 18') forming at least part of an upper end (U) of the expandable stent (10, 10') together with the inlet (I), and
- a plurality of anchors (22; 22', 22") that is arranged in the region of the outlet (O), with a first end (24) of each of the plurality of anchors (22; 22', 22") projecting radially outwardly from the body (16) in the radial direction (B) in the region of the outlet (O) and with the first ends (24) of the plurality of anchors (22; 22', 22") forming at least part of a lower end (L) of the expandable stent (10, 10') together with the outlet.

2. The stent according to claim 1,
wherein the stent frame (12) at least substantially has the shape of an inner bearing race.

3. The stent according to claims 1 or 2,
wherein the height (Hb) of the body (16) is selected in the range 0.3 to 2.0cm, and/or wherein an overall height (H) of the stent (10, 10') of the stent (10, 10') between an uppermost point of the upper end (U) and a lowermost point of the lower end (L) is selected in the range of 0.3 to 2.5cm.

4. The expandable stent in accordance with any one of the preceding claims, wherein the plurality of anchors (22; 22', 22") radially project away from the outlet (O) towards the upper end (U); and/or wherein the plurality of fingers (18; 18') and the plurality of anchors (22; 22', 22') are arranged such that they at least generally project in the same axial direction (A).

5. The expandable stent in accordance with any one of the preceding claims, wherein a second end (26) of the plurality of anchors (22) extends over the height (H_{b}) of the body (16) in the axial direction (A); and/or wherein at least some of the plurality of anchors (22) is configured as respective commissures for the valve member (38) that is longitudinally attached to the plurality of anchors (22), preferably to (the) second ends (26) thereof forming the commissures, over the height (H_{b}) of the body (16) and circumferentially within the body (16) in the region of the inlet (I), so that an upper surface of the valve member (38) is present in the region of the inlet (I) and preferably forms part of the upper end (U).

6. The expandable stent in accordance with any one of the preceding claims, wherein the valve member (38) comprises at least two leaflets (40), and preferably comprises four leaflets (40).

7. The expandable stent in accordance with any one of the preceding claims, further comprising a plurality of elongate struts (14) forming at least part of the body (16) of the stent frame (12), wherein the plurality of elongate struts (14), the plurality of fingers (18; 18') and the plurality of anchors (22; 22', 22") are formed from the same material, such as nitinol, have at least approximately the same thickness in the radial direction (B), and with the plurality of anchors (22; 22', 22") and the plurality of fingers (18; 18') respectively having a greater width than the plurality of elongate struts (14) in the circumferential direction (C) of the body (16).

8. The expandable stent in accordance with any one of the preceding claims, wherein the plurality of fingers (18; 18') form a part of an inlet flange (20) of the expandable stent (10, 10') forming the upper end (U) and/or wherein the plurality of anchors (22; 22', 22") form a part of an outlet flange (20') of the expandable stent (10, 10') forming the lower end (L), and/or wherein the plurality of fingers (18; 18') and the plurality of anchors (22; 22', 22") act as cooperating clamps of the expandable stent (10, 10').

9. The expandable stent in accordance with any one of the preceding claims, wherein the plurality of anchors (22; 22', 22") is formed by or extends from at least some of the material forming the body (16); and/or wherein the plurality of anchors comprise a first group of anchors (22') and a second group of anchors (22"), with a respective anchor of the first group of anchors (22') being shorter than a respective anchor of the second group of anchors (22"); and/or wherein the plurality of fingers (18; 18') is formed by or extends from at least some of the material forming the body (16); and/or wherein the plurality of fingers comprise a first group of fingers and a second group of fingers, with a respective finger of the first group of fingers being shorter than a respective finger of the second group of fingers.

10. The expandable stent in accordance with any one of the preceding claims, wherein the body (16) has an at least generally elliptical shape in a cross-section perpendicular to the axial direction (A), and/or wherein the body (16) has an at least generally cylindrical outer shape over its height (H_{b}) in the axial direction (A), and/or
wherein a diameter (Dᵢ) of the body (16) at the inlet (I) is greater than or equal to a diameter (Dₒ) of the body (16) at the outlet (O) in a cross-section perpendicular to the axial direction (A).

11. The expandable stent in accordance with any one of the preceding claims, further comprising anti-leakage material (28), with the anti-leakage material being arranged to cover at least part of an inner and/or an outer surface (30, 32') of the body (14), preferably with the anti-leakage material (28) being arranged to at least substantially completely cover the inner and/or the outer surface (30, 32') of the body (14), optionally wherein the anti-leakage material (28) at least partly covers the plurality of fingers (18; 18') that form the upper end (U) and/or the plurality of anchors (22; 22', 22") that form the lower end (L).

12. The expandable stent in accordance with any one of the preceding claims, wherein the dry bovine pericardium has a maximum tensile stress selected in the range of 20 to 25 MPa, and/or wherein the rehydrated bovine pericardium has a tensile stress selected in the range of 12 to 15 MPa, and/or wherein a calcium content of the dry pericardium is selected in the range between 0.01 to 0.1 g/kg; and/or wherein the dry pericardium further comprises collagen; and/or the dry pericardium has a thickness selected in the range of 0.3 to 0.5 mm for optimal flexibility and durability; and/or wherein a moisture content of the dry pericardium is less than 1%..

13. A method of assembling an expandable stent comprising the steps of:
(1) providing bovine pericardium as biological tissue as a heart valve;
(2) providing a stent frame;
(3) attaching the heart valve comprising the biological tissue to the stent frame;
(4) testing the functionality of the biological tissue at the stent frame; and
(5) carrying out a method of decellulizing the biological tissue.

14. A method of treating biological tissue comprising the steps of:
(1) soaking of the biological tissue treated with a crosslinking agent with a saline solution;
(2) contacting the soaked biological tissue with an aqueous solution comprising Hydrogen Peroxide;
(3) contacting the biological tissue with an aqueous solution comprising PBS and EDTA;
(4) contacting the biological tissue with a solution comprising glycerol, ethanol and EDTA; and
(5) contacting the biological tissue with a glycerol solution.

15. An expandable stent, in particular the expandable stent according to one of claims 1 to 12 and/or in particular obtainable by the method according to claim 13, comprising a biological tissue prepared by the method according to claim 14.

16. Use of a biological tissue prepared by the method according to claim 16 in an expandable stent according to any one of claims 1 to 14.
